Europäisches Patentamt

European Patent Office

Office européen des brevets

(19)

(11) **EP 0 500 732 B1**

(12) **FASCICULE DE BREVET EUROPEEN**

(45) Date de publication et mention
de la délivrance du brevet:
**16.10.1996 Bulletin 1996/42**

(21) Numéro de dépôt: **90917353.6**

(22) Date de dépôt: **15.11.1990**

(51) Int. Cl.$^6$: **A61K 49/00**

(86) Numéro de dépôt international:
**PCT/FR90/00818**

(87) Numéro de publication internationale:
**WO 91/07191 (30.05.1991 Gazette 1991/12)**

(54) **COMPOSITION DE DIAGNOSTIC COMPRENANT UN COMPLEXE BINUCLEAIRE, SON PROCEDE DE PREPARATION ET SON UTILISATION EN IMAGERIE PAR RESONANCE MAGNETIQUE**

EINEN BINUKLEAREN KOMPLEX ENTHALTENDE DIAGNOSTIKZUSAMMENSETZUNGEN, VERFAHREN ZU DEREN HERSTELLUNG UND VERWENDUNG IN MAGNETISCHER KERNRESONANZBILDERZEUGUNG

DIAGNOSTIC COMPOSITION COMPRISING A BINUCLEAR COMPLEX, A METHOD FOR PREPARING SAME, AND ITS USE IN MAGNETIC RESONANCE IMAGING

(84) Etats contractants désignés:
**AT BE CH DE DK ES FR GB GR IT LI LU NL SE**

(30) Priorité: **16.11.1989 FR 8915035**

(43) Date de publication de la demande:
**02.09.1992 Bulletin 1992/36**

(73) Titulaire: **GUERBET S.A.**
**F-93420 Villepinte (FR)**

(72) Inventeurs:
• **BOURGOIN, Corinne**
**F-75012 Paris (FR)**
• **DESPLANCHES, Gérard**
**F-92100 Boulogne (FR)**
• **LECAYON, Michèle**
**F-91640 Briis-sur-Forge (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
**EP-A- 0 071 564**     **EP-A- 0 133 603**
**EP-A- 0 258 616**     **EP-A- 0 263 059**
**WO-A-87/02893**      **WO-A-89/00052**
**WO-A-90/03804**      **FR-A- 2 539 996**
**FR-A- 2 596 992**

Remarques:
Le dossier contient des informations techniques présentées postérieurement au dépôt de la demande et ne figurant pas dans le présent fascicule.

EP 0 500 732 B1

Printed by Rank Xerox (UK) Business Services
2.13.8/3.4

## Description

La présente invention concerne un procédé de préparation d'une composition comprenant un complexe paramagnétique binucléaire, utilisable pour le diagnostic, en particulier comme agent de contraste en imagerie par résonance magnétique (IRM) ou comme agent de déplacement chimique ou de susceptibilité magnétique.

La spectroscopie par résonance magnétique nucléaire (RMN) a été mise au point en 1946 par Bloch et Purcell, et cette technique a été largement utilisée depuis dans les domaines de la physique, de la chimie organique et de la biochimie, pour étudier la structure chimique des molécules et des groupements moléculaires "in vitro". Le principe de la RMN utilise la conjonction de champs magnétiques et d'ondes de radiofréquence pour mettre en résonance les noyaux de certains atomes. L'un des atomes les plus intéressants étant l'hydrogène, qui est présent dans l'eau constituant la majorité des tissus biologiques (75 à 80% des tissus mous), il a été possible d'envisager l'utilisation de la technique RMN dans le domaine médical, et le développement de l'imagerie par résonance magnétique nucléaire dans ce domaine a connu un essor considérable.

L'image de la distribution des protons est obtenue à partir du signal émis, après mise en résonance des noyaux, et la reconstruction par ordinateur conduit à la visulisation des coupes transverses, sagittales et frontales du corps humain.

Pour obtenir de meilleurs résultats en IRM, on a développé récemment des agents de contraste permettant d'accentue artificiellement le contraste par introduction dans l'organisme à examiner d'éléments modifiant notamment le champ magnétique, par exemple les composés paramagnétiques, afin de perturber les temps de relaxation des noyaux déjà présents dans l'organisme, en ajoutant à la relaxation nucléaire une composante liée à des perturbations par un moment magnétique électronique.

L'utilisation d'agents de contraste de ce type est en particulier décrite dans les documents EP-A- 0 071 564 (Schering A.G.), FR-A- 2 596 992 (Guerbet S.A.) et WO-87/02893 (Board of Regents, the University of Texas System) mais seuls les complexes constitués par le sel de di N-méthyl-glucamine (diméglumine)du complexe Gd(III)-acide diéthylène triamine pentacétique (DTPA) et le sel de lysine du complexe GdIII-acide 1,4,7,10-tétraazacyclododécane-N,N',N'',N'''tétracétique (DOTA) ont été développés respectivement par Schering et Guerbet, et sont utilisés dans les services hospitaliers.

En effet, l'injection "in vivo" de complexes paramagnétiques pose certains problèmes de tolérance biologique.

Ces problèmes qui sont dus notamment aux possibilités d'échange entre l'élément paramagnétique du complexe et les métaux endogènes du sujet auquel le complexe a été injecté, se traduisent, d'une part, par la libération "in vivo" de l'élément paramagnétique, plus ou moins toxique, du complexe injecté et, d'autre part, par un appauvrissement en métal endogène, par exemple en calcium ou en zinc, du sujet.

Pour éviter ces problèmes, deux solutions ont été proposées qui consistent :

- soit à ajouter un sel de calcium, c'est-à-dire un sel de métal endogène, à la solution de complexe injecté pour éviter l'appauvrissement en métal endogène, comme il est décrit dans WO-89/00052,
- soit à ajouter à la solution de complexe paramagnétique, un complexe formé entre le même ligand et le calcium pour éviter le relargage de l'élément paramagnétique comme il est décrit dans WO-9003804.

Ces solutions ne sont toutefois pas entièrement satisfaisantes car l'osmolalité des compositions reste élevée, ces deux additifs n'apportant aucune amélioration sur l'osmolalité du complexe.

Or, comme il est indiqué dans Radiology 1988, 166, 897-899, l'osmolalité du complexe est un facteur déterminant dans la toxicité des complexes paramagnétiques.

Ainsi, le complexe constitué par le sel de di-N-méthyl-glucamine du DTPA-Gd(III) présente une pression osmotique élevée (1940mosm/kg selon les indications de Berlex Lab. qui commercialise ce produit sous la référence Magnevist®) ; de plus, sa viscosité est importante et on doit l'utiliser en concentrations élevées, généralement sous la forme d'une solution à 0,5mol/l de complexe. Aussi, sa toxicité reste élevée.

En outre, EP-A-258 616 décrit des sels de calcium, ou magnésium de chélates de poly(acide-alkylèneamino)alkanes avec des cations paramagnétiques, préparés par addition de sels minéraux ou organiques de calcium ou magnésium dans une solution du chélate.

FR-2 539 996 décrit des sels d'un anion provenant d'un acide complexant un ion central métallique. Lorsque ces sels comportent des atomes d'hydrogène acides libres, ceux-ci peuvent être à leur tour salifiés par des cations minéraux ou organiques de façon à faciliter leur purification.

EP-A-71564 décrit aussi des sels de complexes paramagnétiques d'acides aminopolycarboxyliques préparés par action d'une base, telle que la N-méthylglucamine, sur les groupes acides libres du complexe.

EP-A-263059 décrit des sels minéraux ou organiques d'autres acides aminopolycarboxyliques dont une fonction carboxylique est sous la forme d'amide, pouvant être utilisés comme complexants d'ions paramagnétiques.

EP-A-133603 décrit des complexes paramagnétiques de structures variées comme agents de contraste en résonance paramagnétique nucléaire dont le sel mixte de gadolinium et de calcium du DTPA, lequel est obtenu par action de l'oxyde de Gd sur le DTPA suivie de celle de $CaCO_3$.

WO 89/00052 a trait à un procédé de préparation d'agents de contraste pour l'imagerie RMN consistant à ajouter des ions calcium à un chélate obtenu par complexation d'un anion dérivé d'un acide complexant avec un cation métallique central.

WO 90/03804 concerne une composition de diagnostic renfermant à la fois un chélate du bisamide-DTPA avec un on métallique paramagnétique et un chélate du bisamide-DTPA avec le calcium.

La présente invention vise à fournir une composition de diagnostic utilisable en IRM qui pallie les inconvénients des compositions décrites ci-dessus.

La composition de diagnostic préparée selon le procédé de l'invention comprend un complexe binucléaire entre un ligand polyaminopolycarboxylique cyclique ou acyclique comportant au moins 6 atomes donneurs d'électrons, un métal paramagnétique choisi parmi les lanthanides et les métaux de transition, et un métal endogène choisi parmi Ca et Zn.

Plus précisément, l'invention concerne un procédé de préparation d'une composition aqueuse de diagnostic par résonance magnétique nucléaire comprenant un complexe mononucléaire formé entre un on paramagnétique choisi parmi les lanthanides et les métaux de transition et un ligand polyaminocarboxylique, cyclique ou acyclique, comportant au moins six atomes donneurs d'électrons, et un complexe binucléaire formé avec le même ion paramagnétique, le même ligand et l'ion calcium ou zinc, caractérisé en ce qu'il comprend les étapes suivantes :

a) préparation du complexe mononucléaire par réaction du ligand polyaminocarboxylique avec l'oxyde du métal paramagnétique;

b) préparation du complexe binucléaire par réaction du ligand polyaminocarboxylique avec l'oxyde du métal paramagnétique puis avec le carbonate de calcium ou de zinc;

c) mélange en solution des complexes obtenus aux étapes (a) et (b) suivi d'une salification avec la N-méthylglucamine ou un aminoacide basique.

Dans l'invention, on entend par complexe binucléaire, bimétallique ou hétéronucléaire, un complexe dans lequel l'élément central du complexe est formé de deux métaux différents qui sont l'élément paramagnétique (lanthanide ou métal de transition) et le métal endogène.

Un tel complexe binucléaire se comporte comme une particule unique et présente de ce fait une osmolalité plus faible que celle d'un complexe mononucléaire formé entre un élément paramagnétique et un ligand comportant au moins 6 atomes donneurs d'électrons.

De plus, avec ce complexe binucléaire, on limite lors de l'injection "in vivo", la captation par le complexe des métaux endogènes ainsi que la libération "in vivo" de l'élément paramagnétique toxique.

En effet, l'échange d'un métal endogène provenant du sujet se produit "en priorité" avec le métal endogène présent dans l'élément central du complexe binucléaire.

Ainsi, l'utilisation d'un tel complexe binucléaire dans la composition de diagnostic permet de surmonter les inconvénients des solutions connues et de réduire également l'osmolalité de la solution injectée.

La composition de diagnostic comprend en outre un complexe mononucléaire entre le même ligand et le même métal paramagnétique.

Dans ce mélange, le rapport molaire du complexe mononucléaire au complexe binucléaire peut être de 0,1 à 1.

Un tel mélange même avec un rapport 1:1 permet de diminuer suffisamment l'osmolalité et la toxicité de la composition de diagnostic pour la rendre plus adaptée à l'injection "in vivo" et permettre son utilisation comme agent de contraste ou comme agent de déplacement chimique ou de susceptibilité magnétique en IRM.

Dans l'invention, le métal paramagnétique peut être choisi parmi les lanthanides et les métaux de transition. On peut utiliser par exemple le gadolinium, le manganèse, le fer ou le cuivre, dans les compositions destinées à servir d'agent de contraste.

Dans le cas où la composition est destinée à servir d'agent de déplacement chimique ou de susceptibilité magnétique, on préfère utiliser comme métal paramagnétique Tm, Tb, Gd, Eu, Dy, Ho ou Er.

Les métaux endogènes utilisés sont ceux qui sont généralement présents à l'état de traces mais constituent des éléments essentiels du corps humain. De préférence, on utilise Ca ou Zn.

Les ligands polyaminopolycarboxyliques utilisés dans l'invention doivent comporter au moins 6 atomes donneurs d'électrons et peuvent être des ligands cycliques ou acycliques.

Parmi ces ligands, on peut citer en particulier les acides aminopolycarboxyliques tels que ceux décrits dans EP-A-0071564 par exemple l'acide diéthylène triamine pentacétique (DTPA), l'acide triéthylène tétramine hexacétique (TTHA), l'acide trans 1,2-diaminohexane tétracétique (CYDTA) et les composés tétraaza ou diazamacrocycliques comme l'acide 1,4,7,10-tétraazacyclododécane N, N', N'', N''' tétracétique (DOTA). Le ligand peut aussi être couplé à une molécule biologique telle qu'un anticorps monoclonal.

La solution aqueuse est une solution physiologiquement acceptable qui peut comprendre d'autres additifs pharmaceutiquement acceptables tels que des stabilisants, si nécessaire.

Généralement, la solution comprend de 0,2mol/l à 1,2mol/l au total de complexes du métal paramagnétique.

Les compositions peuvent être préparées facilement à partir d'un composé du métal paramagnétique, par exemple un oxyde, du ligand approprié, d'un sel du métal endogène et éventuellement dune base organique telle que les aminoacides basiques et la N-méthylglucamine.

Selon un mode de réalisation préféré, on prépare une composition aqueuse de diagnostic comprenant l'ion gadolinium comme on métallique paramagnétique en mettant en oeuvre les étapes suivantes :

a) préparation d'un complexe mononucléaire du DTPA (acide diéthylène triamine pentaacétique) avec l'ion $Gd^{3+}$ par réaction du DTPA avec le $Gd_2O_3$;

b) préparation d'un complexe binucléaire du DTPA avec l'on $Gd^{3+}$ et l'ion calcium par réaction du DTPA avec $Gd_2O_3$ puis avec le carbonate de calcium, et

c) mélange en solution des complexes mononucléaire et binucléaire obtenus aux étapes (a) et (b), suivi d'une salification avec la N-méthylglucamine ou un aminoacide basique.

Lorsque les complexes formés possèdent des charges négatives, on réalise une étape complémentaire de neutralisation par addition à la solution aqueuse obtenue dans l'étape c) d'une base organique choisie parmi les aminoacides basiques et la N-méthylglucamine.

De manière préférée, la salification est effectuée avec la N-méthylglucamine.

A titre d'exemple d'aminoacide utilisable, on peut citer la lysine et l'arginine.

De préférence, dans l'étape c), le rapport molaire du complexe mononucléaire / complexe binucléaire est de 0,1 à 1.

Les compositions de diagnostic qui sont administrables à l'homme, peuvent être utilisées en particulier comme agent de contraste en imagerie par résonance magnétique (IRM).

Elles peuvent aussi être utilisées comme agent de déplacement chimique ou de susceptibilité magnétique en imagerie par résonance magnétique, comme il est décrit dans Mag Res in Med. 6, 164-174, 1988. Dans ce cas le métal paramagnétique est de préférence Tm, Tb, Gd, Eu, Dy Ho ou Er.

Les compositions de diagnostic ainsi obtenues peuvent être administrées à l'homme par voie parentérale (notamment vasculaire ou lymphatique), sous arachnoïdienne, orale ou intrabronchique.

Pour l'administration par voie parantérale, ou orale, la composition est de préférence constituée par une solution du mélange de complexes mono et binucléaires dans un solvant aqueux physiologiquement acceptable qui contient de préférence de 0,2 à 1,2 mol/l de complexes paramagnétiques, par exemple 0,5mol/l.

La solution contient notamment 0,8mol/l des complexes et la dose administrable est généralement de 0,05 à 5 mmol/kg de poids corporel, par exemple 0,1 mmol/kg.

L'invention sera mieux comprise à la lecture de la description suivante donnée bien entendu à titre illustratif, qui concerne la préparation conformément au procédé de l'invention et les propriétés d'une composition de diagnostic comprenant un mélange d'un complexe mononucléaire (Gd) et d'un complexe binucléaire (Gd-Ca).

I. Préparation de la composition de diagnostic.

a) Préparation du complexe mononucléaire Gd-DTPA.

On dissout 100g (0,275mol) d'oxyde de gadolinium $Gd_2O_3$ et 217g (0,55 mol) d'acide diéthylène triamine pentacétique (DTPA) dans 2l d'eau pour préparations injectables (ppi). On chauffe au reflux jusqu'à dissolution complète des produits, on filtre la solution et on mesure exactement le volume de solution recueilli pour calculer la concentration en complexe $H_2Gd.DTPA$. Cette concentration est de 0,44mol/l.

b) Préparation du complexe binucléaire Ca-Gd(III)-DTPA.

On dissout 100g (0,275mol) d'oxyde de gadolinium $Gd_2O_3$ et 217g (0,55mol) d'acide diéthylène triamine pentacétique (DTPA) dans 2l d'eau (ppi), on chauffe à reflux jusqu'à dissolution complète des produits, on filtre la solution, puis on chauffe en ajoutant 55,23g (0,55mol) de carbonate de calcium $CaCO_3$. On réduit le volume de la solution de 1/4 en chauffant. On recueille le précipité que l'on sèche sous vide, puis on le redissout dans 2l d'eau (ppi), on réduit le volume de la solution de 1/4 par chauffage, on recueille le précipité et on le sèche sous vide.

On obtient ainsi 279,6g (0,477mol) de Ca-Gd-DTPA.

c) <u>Préparation de la solution aqueuse des complexes Gd-DTPA et Ca-Gd-DTPA</u>.

On mélange 1074ml de la solution de $H_2$Gd-DTPA obtenue en a) avec 1311ml d'eau, puis on dissout dans la solution obtenue 279,6g du complexe Ca-Gd-DTPA obtenu dans l'étape b) et on mélange la solution jusqu'à dissolution complète du produit.

d) <u>Neutralisation</u> .

On ajuste le pH de la solution obtenu dans l'étape c) à 7 en ajoutant 150g de N-méthyl glucamine.
On obtient ainsi une solution de Gd-DTPA et Gd-Ca-DTPA.
On stérilise alors la solution par filtration et on la distribue dans des flacons, à raison de 20ml par flacon, en réalisant cette opération dans une enceinte stérile.

## II. Formulation de la solution de complexes.

- <u>Complexes</u>

  teneur par flacon de 20ml : 6,08g
  teneur par ml : 0,304g

- <u>gadolinium</u>

  teneur par flacon de 20ml : 1,26g
  teneur par ml : 0,063g

- <u>calcium</u>

  teneur par flacon de 20ml : 0,16g
  teneur par ml : 0,008g

- <u>acide diéthylène triamine pentacétique (DTPA)</u>

  teneur par flacon de 20ml : 3,10g
  teneur par ml : 0,155g

- <u>N-méthylglucamine</u>

  teneur par flacon de 20ml : 1,56g
  teneur par ml : 0,078g.

Les caractéristiques physicochimiques de la solution, sa stabilité, sa toxicité aigüe, sa capacité de liaison aux protéines plasmatiques et les temps de relaxation $T_1$ et $T_2$ dans différents organes sont donnés ci-dessous.

## 1. Caractéristiques physicochimiques.

- <u>Osmolalité</u> déterminée au moyen d'un osmomètre par la technique propharmacopoea 360, Ordre National des Pharmaciens n° 293, juin 1986 :

  osmolalité = 812 mosm/kg à 37°C.

- <u>Viscosité</u> déterminée au moyen d'un tube capillaire selon les normes de la pharmacopée française :

  viscosité = 0,096 cPas.

## 2. Stabilité de la composition.

On a étudié cette stabilité en milieu sanguin en suivant l'évolution de la concentration en gadolinium libre et en calcium libre par chromatographie sur papier avec des complexes marqués au gadolinium 153 et au Ca 47.

## EP 0 500 732 B1

Les résultats obtenus sont donnés dans le tableau 1 qui suit.

TABLEAU 1

| Stabilité du complexe DTPA-Gd-Ca (méglumine) | | | | |
|---|---|---|---|---|
| SOLUTION DE COMPLEXE | SANG | | SOLUTION DE GLUCOSE | URINE |
| | Plasma | Hématies | | |
| $^{153}$Gd complexé | 99,05% | $^{153}$Gd fixé par les hématies 0,001% | | $<2,5.10^{-8}$g/l |
| $^{153}$Gd libre | 0,95% | | 0,85% | |
| $^{47}$Ca complexé | 98,9% | $^{47}$Ca fixé par les hématies 0,7% | | |
| $^{47}$Ca libre | 0,4% | | 6,4% | |
| Gd et Ca restent complexés en solution dans le plasma en présence d'hématies. | | | | |

On teste également la présence de gadolinium libre dans les urines "in vivo". On obtient pour tous les échantillons d'urine une coloration verte et la méthode colorimétrique ne met pas en évidence de gadolinium libre, la teneur en gadolinium libre étant inférieure à $2,5.10^{-8}$g/l.

### 3. Toxicité aigué de la composition

On teste la toxicité aigué de la composition chez la souri mâle par injection intraveineuse unique en 1 min, en utilisant la méthode de Bliss. Les résultats obtenus sont les suivants :

- DL50=3760,6mg/ g (5,02mmol/kg).

Cette valeur est supérieure à celle que l'on obtient avec le complexe DTPA-Gd diméglumine du document FR-A-2 596 992 (4,45 mmol/kg). Ainsi, la composition préparée conformement au procédé de l'invention est moins toxique.

### 4. Capacité de liaison aux protéines plasmatiques.

On n'observe pas de liaison protéique dans le plasma pour les complexes testés en utilisant le chlorure de gadolinium 153 comme témoin.

### 5. Evaluation des temps de relaxation T1 et T2 dans les organes.

Pour cette détermination, on utilise des rats mâles de souche Wistar pesant environ 250g et l'on injecte à la moitié des rats une solution d'urée à 3mol/l dans la carotide droite pour rompre la barrière hématoencéphalique (BHE), puis la composition de l'invention correspondant à la formulation décrite en II à une dose de 2 ml/kg par voie intraveineuse.
1 minute après, on tue les rats, on prélève leur cerveau et on détermine les temps de relaxation T1 et T2 dans l'hémisphère gauche, l'hémisphère droit et le cervelet.
Le temps de relaxation longitudinale T1 (Spin-réseau) est lié à la création de l'aimantation longitudinale axée sur le champ magnetique Bo et il est déterminé par les relations énergétiques entre les noyaux et le réseau.
Le temps de relaxation transversale T2 (Spin-spin) est défini par le retour à l'équilibre en grandeur et en orientation de l'aimantation longitudinale (paralléle à Bo) qui a été annulée ou modifiée par l'impulsion de radiofréquence. On l'exprime aussi par le retour à zéro de l'aimantation transversale qui a été créée par l'impulsion de radiofréquence.
Les résultats obtenus sont donnés dans le tableau 2 annexé.
Ces résultats montrent que de 1min à 30min après l'injection, les temps de relaxation T1 et T2 sont raccourcis, ce qui prouve que l'agent de contraste est efficace.
Ainsi, par rapport au complexe Gd-DTPA diméglumine, les avantages de la composition préparée selon le procédé de l'invention sont les suivants :

- une pression osmotique plus faible,
- une viscosité plus faible,
- une toxicité plus faible, et

6

- la possibilité d'être utilisé à une concentration plus faible.

Par ailleurs, elle possède une très grande stabilité due à la complexation du calcium.

En effet, cette composition comprend moins de DTPA libre que le complexe de Shering Gd-DTPA-diméglumine, qui peut capter du calcium dans le sang donc produire une déminéralisation due au DTPA libre.

Dans le cas de l'invention, le complexe binucléaire qui contient déjà du calcium complexé, ne complexera pas le calcium de l'organe.

De la même façon, on peut obtenir le complexe binucléaire DTPA-Gd-Zn.

TABLEAU 2

| TEMPS DE RELAXATION DANS LE CERVEAU | | | | |
|---|---|---|---|---|
| | | Animal sain sans injection de produit de contraste | Animal BHE rompue, sacrifice 1 minute, après injection de DTPA-Gd-Ca (méglumine) | |
| | | Millisecondes | Millisecondes | % de diminution |
| T1 | Hémisphère gauche | 629,32 ms | 344,42 ms | 45,2 |
| | Hémisphère droit | 689,72 ms | 302,32 ms | 56,2 |
| | Cervelet | 655,78 ms | 291,04 ms | 55,6 |
| T2 | Hémisphère gauche | 81,75 ms | 70,05 ms | 14,4 |
| | Hémisphère droit | 81,63 ms | 65,72 ms | 19,5 |
| | Cervelet | 88,60 ms | 69,70 ms | 21,6 |

## Revendications

1. Procédé de préparation d'une composition aqueuse de diagnostic par résonance magnétique nucléaire comprenant un complexe mononucléaire formé entre un ion paramagnétique choisi parmi les lanthanides et les métaux de transition et un ligand polyaminocarboxylique, cyclique ou acyclique, comportant au moins six atomes donneurs d'électrons, et un complexe binucléaire formé avec le même ion paramagnétique, le même ligand et l'ion calcium ou zinc, caractérisé en ce qu'il comprend les étapes suivantes :

   a) préparation du complexe mononucléaire par réaction du ligand polyaminocarboxylique avec l'oxyde du métal paramagnétique;
   b) préparation du complexe binucléaire par réaction du ligand polyaminocarboxylique avec l'oxyde du métal paramagnétique puis avec le carbonate de calcium ou de zinc;
   c) mélange en solution des complexes obtenus aux étapes (a) et (b) suivi d'une salification avec la N-méthylglucamine ou un aminoacide basique.

2. Procédé selon la revendication 1, caractérisé en ce que le rapport molaire dudit complexe mononucléaire audit complexe binucléaire est de 0,1 à 1.

3. Procédé selon l'une des revendications 1 et 2, dans laquelle l'on paramagnétique est le gadolinium, caractérisé en ce qu'il comprend les étapes suivantes :

   a) préparation d'un complexe mononucléaire du DTPA (acide diéthylène triamine pentaacétique) avec l'ion $Gd^{3+}$ par réaction du DTPA avec le $Gd_2O_3$;
   b) préparation d'un complexe binucléaire du DTPA avec l'ion $Gd^{3+}$ et l'ion calcium par réaction du DTPA avec $Gd_2O_3$ puis avec le carbonate de calcium, et
   c) mélange en solution des complexes mononucléaire et binucléaire obtenus aux étapes (a) et (b), suivi d'une salification avec la N-méthylglucamine ou un aminoacide basique.

4. Procédé selon la revendication 3, caractérisé en ce que la salification est effectuée avec la N-méthylglucamine.

## Claims

1. Method of preparing an aqueous composition for diagnosis by magnetic resonance imaging comprising a mononuclear complex formed between a paramagnetic ion selected from among the lanthanides and the transitional metals and a polyaminocarboxylic cyclic or acyclic ligand, comprising at least six electron-supplying atoms, and a binuclear complex formed with the same paramagnetic ion, the same ligand and the calcium or zinc ion, characterised in that it comprises the following stages:

   a) preparation of the mononuclear complex by reaction of the polyaminocarboxylic ligand with the oxide of the paramagnetic metal;

   b) preparation of the binuclear complex by reaction of the polyaminocarboxylic ligand with the oxide of the paramagnetic metal, then with calcium carbonate or zinc;

   c) mixture in solution of the complexes obtained in stages a) and b) followed by salinification with N-methylglucamine or a basic amino acid.

2. Method according to claim 1, characterised in that the molar ratio of the said mononuclear complex to the said binuclear complex is 0.1 to 1.

3. Method according to one of claims 1 and 2, in which the paramagnetic ion is gadolinium, characterised in that it comprises the following stages:

   a) preparation of a mononuclear complex of DTPA (diethylene triamine pentaacetic acid) with the ion $Gd^{3+}$ by reaction of the DTPA with $Gd_2O_3$;

   b) preparation of a binuclear complex of DTPA with the $Gd^{3+}$ ion and the calcium ion by reaction of DTPA with $Gd_2O_3$, then with calcium carbonate, and

   c) mixture in solution of the mononuclear and binuclear complexes obtained in stages a) and b), followed by salinification with N-methylglucamine or a basic amino acid.

4. Method according to claim 3, characterised in that salinification is effected with N-methylglucamine.

## Patentansprüche

1. Verfahren zur Herstellung einer wäßrigen Zusammensetzung für die kernmagnetische Resonanzdiagnose, umfassend einen mononuklearen Komplex, der aus einem paramagnetischen Ion ausgewählt aus Lanthaniden und Übergangsmetallen und einem cyclischen oder acyclischen Polyaminocarboxyl-Liganden, der mindestens sechs Elektronendonor-Atome aufweist, gebildet ist, und einem binuklearen Komplex, der aus dem gleichen paramagnetischen Ion, dem gleichen Liganden und Calcium- oder Zinkionen gebildet ist, **dadurch gekennzeichnet**, daß es die folgenden Stufen umfaßt:

   a) Herstellung des mononuklearen Komplexes durch Reaktion des Polyaminocarboxyl-Liganden mit dem Oxid des paramagnetischen Metalls;
   b) Herstellung des binuklearen Komplexes durch Reaktion des Polyaminocarboxyl-Liganden mit dem Oxid des paramagnetischen Metalls und dann mit Calcium- oder Zinkcarbonat;
   c) Vermischen der in den Stufen (a) und (b) erhaltenen Komplexe in Lösung, gefolgt von einer Salzbildung mit N-Methylglucamin oder einer basischen Aminosäure.

2. Verfahren nach Anspruch 1, **dadurch gekennzeichnet**, daß das Molverhältnis des mononuklearen Komplexes zu dem binuklearen Komplex 0,1 bis 1 beträgt.

3. Verfahren nach einem der Ansprüche 1 und 2, worin das paramagnetische Ion das von Gadolinium ist, **dadurch gekennzeichnet**, daß es die folgenden Stufen umfaßt:

   a) Herstellung eines mononuklearen Komplexes von DTPA (Diethylentriaminpentaessigsäure) mit dem $Gd^{3+}$-Ion durch Reaktion von DTPA mit $Gd_2O_3$;

b) Herstellung eines binuklearen DTPA-Komplexes mit dem $Gd^{3+}$-Ion und dem Calciumion durch Reaktion von DTPA mit $Gd_2O_3$ und dann mit Calciumcarbonat und

c) Vermischen der in den Stufen (a) und (b) erhaltenen mononuklearen und binuklearen Komplexe in Lösung, gefolgt von einer Salzbildung mit N-Methylglucamin oder einer basischen Aminosäure.

4. Verfahren nach Anspruch 3, **dadurch gekennzeichnet**, daß die Salzbildung mit N-Methylglucamin bewirkt wird.